(19) Europäisches Patentamt
European Patent Office
Office européen des brevets

(11) Veröffentlichungsnummer: **0 001 061**
**B1**

(12) **EUROPÄISCHE PATENTSCHRIFT**

(45) Veröffentlichungstag der Patentschrift: **01.04.81**

(21) Anmeldenummer: **78100685.3**

(22) Anmeldetag: **17.08.78**

(51) Int. Cl.³: **C 07 C 103/52,**
A 61 K 37/02

(54) **Neue Polypeptide, ihre Herstellung und Heilmittel, die diese Polypeptide enthalten.**

(30) Priorität: **07.09.77 CH 10939/77**
**17.11.77 CH 14058/77**
**19.05.78 CH 5468/78**

(43) Veröffentlichungstag der Anmeldung:
**21.03.79 Patentblatt 79/6**

(45) Bekanntmachung des Hinweises auf die Patenterteilung:
**01.04.81 Patentblatt 81/13**

(84) Benannte Vertragsstaaten:
**BE CH DE FR GB LU NL SE**

(56) Entgegenhaltungen:
**FR - A - 2 339 590**

(73) Patentinhaber: **SANDOZ AG**
**Lichtstrasse 35**
**CH-4002 Basel (CH)**

(72) Erfinder: **Pless, Janos, Dr.**
**Kluserstrasse 24**
**CH-4053 Basel (CH)**
Erfinder: **Sandrin, Edmond**
**Weilstrasse 78**
**CH-4125 Riehen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 001 061 B1

# Neue Polypeptide, ihre Herstellung und Heilmittel, die diese Polypeptide enthalten

Gegenstand der Erfindung sind neue Polypeptide bzw. Polypeptidderivate der Formel

$$A — B — C — D — E \qquad I$$

worin

A für H-Trp, H-MeTrp, H-Trp(5-OH) oder H-MeTrp(5-OH)

B für Ala,

C für Ser, Thr, Ala, Gly, Val oder einen Rest der Formel

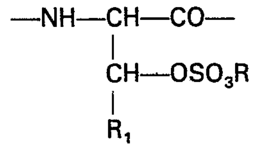

oder

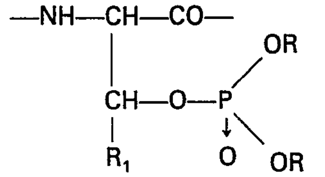

worin

R Wasserstoff, Alkyl mit 1—4 C-Atomen oder ein Alkali- oder Erdalkalimetall und

$R_1$ Wasserstoff oder Methyl bedeuten,

D für Gly oder Sar,

E für den Rest

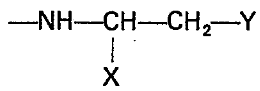

X und Y unabhängig voneinander für COZ oder $CH_2OH$,

Z für OH, $OR_2$, $NH_2$, $NHR_3$,

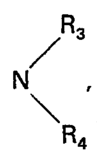

,

$R_2$, $R_3$ und $R_4$ unabhängig voneinander für Alkylgruppen mit 1—4 C-Atomen stehen, wobei die Reste A, B, C und E die Konfiguration der L-, D- oder D,L-Reihe haben können, sowie Salze, Säureadditionssalze und Komplexe dieser Polypeptide bzw. Polypeptidderivate und Verfahren zur Herstellung dieser Verbindungen.

Als Säureadditionssalze kommen solche mit organischen Säuren, polymeren Säuren und Salze mit anorganischen Säuren in Frage. Unter den Komplexen sind z.B. anorganische Verbindungen, die sich von Metallen wie Calcium, Magnesium, Aluminium, Cobalt und insbesondere von Zink ableiten lassen, zu nennen.

Die Erfindung umfasst Verfahren zur Herstellung von Verbindungen der obigen Formel. Sie können analog zu den für die Synthese von Verbindungen dieser Art allgemein bekannten Methoden hergestellt werden.

Beispielsweise können die Verbindungen der obigen Formel hergestellt werden,

a) indem man mindestens eine Schutzgruppe, die in einer geschützten Verbindung mit der in Formel I angegebenen Sequenz vorhanden ist, entfernt, oder

b) indem man zwei Peptideinheiten, von denen jede mindestens eine Aminosäure oder einen Aminoalkohol in geschützter oder ungeschützter Form enthält, durch eine Amidbindung miteinander verknüpft, wobei die Peptidbindung in der Weise erfolgen soll, dass die in der Formel I enthaltene Aminosäuresequenz hergestellt wird und anschliessend gegebenenfalls die Verfahrensstufe a) ausgeführt wird, oder

c) indem man eine Gruppe E in eine andere Gruppe E mit der oben angeführten Definition überführt, wobei eine ungeschützte oder geschützte Verbindung der Formel I erhalten wird und in letzterem Fall der Verfahrensschritt a) ausgeführt wird.

Insofern die Herstellung der Ausgangsprodukte nicht besonders beschrieben wird, sind diese Verbindungen bekannt oder können nach an sich bekannten Methoden hergestellt und gereinigt werden. Ebenso können diese analog zu den in den Beispielen beschriebenen Verfahren hergestellt werden.

Die Polypeptide bzw. Polypeptidderivate der Formel I und die physiologisch verträglichen Säureadditionssalze bzw. Komplexe dieser Verbindungen weisen im Tierversuch interessante pharmakodynamische Eigenschaften auf. Sie können daher als Heilmittel verwendet werden. Insbesondere besitzen sie eine ZNS-Aktivität. Diese Wirkung, die, abhängig von der Struktur der Verbindungen sowie von deren Dosierung, sowohl ZNS-dämpfend als auch ZNS-stimulierend sein kann, wird z.B. durch die Aenderungen im spontanen Verhalten von Mäusen und Ratten nach oralem oder i.p. Verabreichen der erfindungsgemässen Verbindungen nachgewiesen. Das Prinzip dieser Methode (P.O.T.) wurde von S. Irwin (Gordon Research Conference, Medicinal Chemistry, 1959), von J.H. Nodide und P.E. Siegler (Animal and clinical pharmacologic techniques in drug evaluation, Chicago 1964) sowie in Psychopharmacologia *13*, 222—257 (Berlin, 1968) beschrieben.

Diese Wirkungen konnten auch anhand des Motrontests bei Mäusen gezeigt werden. In diesem Test wird die motorische Aktivität elektronisch mit Hilfe eines Motilitäts-Messgeräts bestimmt. Pro Dosis werden 2 × 5 Mäuse (als Kontrollgruppe bzw. mit Substanz) eingesetzt und deren Laufaktivität sowie das Aufrichten separat alle 15 Minuten über eine Versuchsdauer von 75 Minuten bestimmt. Die ED 50 bzw. die ED 200 ist die Dosis, bei der die motorische Aktivität der Mäuse halb bzw. doppelt so gross wie bei den Kontrolltieren ist. Da viele sedierende Substanzen bei der Maus sowohl hemmend wie stimulierend wirken können, ermöglicht diese Art der Auswertung eine zeitliche und quantitative Differenzierung dieser beiden entgegengesetzten Wirkungen auf die motorische Aktivität.

Die erfindungsgemässen Verbindungen besitzen auch antidepressive Eigenschaften. beispielsweise hemmen die Verbindungen die durch Tetrabenazin erzeugte Katalepsie und Ptosis in Ratten in einer Versuchsmethode nach Stille (Arzneimittelforschung 1964, *14*, 534).

Anhand dieser pharmakologischen Versuche konnte festgestellt werden, dass die nachfolgenden Verbindungen der Formel

$$A' — B — C' — D — E \qquad II$$

worin

A′ für H-Trp und

C′ für Gly, Ser,

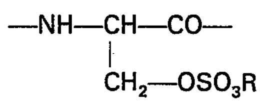

oder

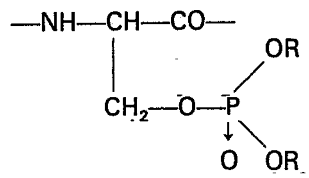

stehen,

hauptsächlich ZNS-dämpfend wirken, während die sonstigen Verbindungen der Formel I eher ZNS-stimulierend sind.

Aufgrund der beobachteten pharmakologischen Eigenschaften können die Verbindungen mit dämpfender Wirkung als Sedativa und insbesondere als Schlafmittel, die Verbindungen mit stimulierender Wirkung bei der Behandlung der Cerabralinsuffizienz oder bei depressiven Zustanden verwendet werden.

Die zu verwendenden Dosen variieren naturgemäss je nach Art der Substanz, der Administration und des zu behandelnden Zustandes. Zufriedenstellende Resultate erhält man bei Verabreichung von Verbindungen der Formel I in einer Dosis von 0,1 bis 100 mg/kg Tierkörpergewicht.

Bei grösseren Säugetieren ist eine täglich zu verabreichende Menge zwischen 5 und 500 mg angezeigt. Diese Dosis kann gegebenenfalls auch in kleineren Dosen 2 bis 4 mal täglich oder in Retardform verabreicht werden. Eine Einheitsdosis, beispielsweise eine zur oralen Verabreichung geeignete Tablette, kann zwischen 1,25 und 250 mg des Wirkstoffes zusammen mit geeigneten pharmazeutisch indifferenten Hilfsstoffen enthalten.

Die Erfindung betrifft auch Heilmittel, die eine Verbindung der Formel I enthalten. Diese Heilmittel, beispielsweise eine Lösung oder eine Tablette, können nach bekannten Methoden, unter Verwendung der üblichen Hilfs- und Trägerstoffe, hergestellt werden.

In einer Gruppe der erfindungsgemässen Verbindungen bedeuten

A H-Trp, H-MeTrp, H-Trp(5-OH) oder H-MeTrp(5-OH),

B Ala,

C Ser, Thr, Ala, Gly oder einen Rest der Formel

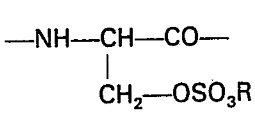

oder

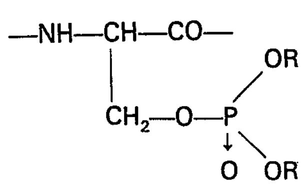

worin R für Wasserstoff, Alkyl mit 1—4 C-Atomen oder ein Alkali- oder Erdalkalimetall steht,
D Gly und
E Asp-OH, Asp-OR', Asp-$NH_2$, Asn-OH, Asn-OR', Asn-$NH_2$, Asparaginol oder den Rest

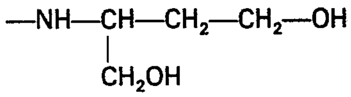

wobei R' eine Alkylgruppe mit 1—4 C-Atomen bedeutet und die Reste A, B, C und E die Konfiguration der L-, D- oder D,L-Reihe haben können.

In einer zweiten Gruppe bedeuten
A H-Trp, H-MeTrp, H-Trp(5-OH) oder H-MeTrp(5-OH),
B Ala,
C Ser, Thr, Ala, Gly oder einen Rest der Formel

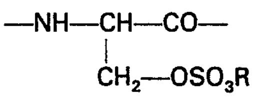

oder

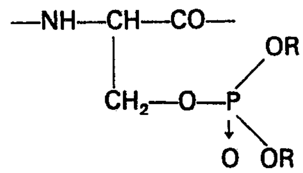

worin R Wasserstoff, Alkyl mit 1—4 C-Atomen oder ein Alkali- oder Erdalkalimetall bedeutet,
D Gly und
E Asp(OR')—X'
R' eine Alkylgruppe mit 1—4 C-Atomen und
X' eine Hydroxylgruppe, eine Alkoxygruppe mit 1—4 C-Atomen oder eine $NH_2$-Gruppe,
wobei die Reste A, B, C und Asp(OR')—X' die Konfiguration der L-, D- oder D,L-Reihe haben können.

In dem folgenden Beispiel erfolgen alle Temperaturangaben in Celsiusgraden.

Es werden folgende Abkürzungen verwendet:

| | | |
|---|---|---|
| Asn | = | Asparaginrest |
| Asn-ol | = | Asparaginolrest |
| Asp-diol | = | Asparagindiol- = 2-amino-butan-1,4-diolrest |
| Asp($NHCH_3$)-ol | = | N'-Methylasparaginolrest |
| Asp(OMe)-OMe | = | Asparaginsäuredimethylesterrest |
| DMF | = | Dimethylformamid |
| Et | = | Aethyl |
| Me | = | Methyl |
| MeTrp | = | N-Methyltryptophanrest |
| (5-OH)-Trp | = | 5-Hydroxytryptophanrest |
| OTcp | = | 2,4,5-Trichlorphenoxy |
| TFA | = | Trifluoressigsäure |
| Z | = | Benzyloxycarbonyl |

Alle Aminosäurereste ausser Glycyl sowie alle Aminoalkoholreste haben, sofern nicht anders angegeben, die L-Konfiguration. Ein Aminoalkohol wird der L-Reihe zugeordnet, wenn seine $CH_2OH$-Gruppe die Stelle der $\alpha$-COOH-Gruppe in der entsprechenden L-Aminosäure einnimmt.

### Beispiel 1

*H-Trp-Ala-Ser-Gly-L-Asn-ol*

Zu einer Lösung von 2,0 g Z-Trp-Ala-Ser-Gly-L-Asn-ol in 80 ml Dioxan und 3.2 ml wässriger 1N—HCl gibt man 1 g eines Pd-Katalysators und hydriert bei Raumtemperatur unter normalem Druck, bis kein Wasserstoff mehr aufgenommen wird. Man filtriert vom Katalysator ab, dampft das Lösungsmittel ab und trituriert den Rückstand mit Aether. Man erhält das Hydrochlorid der Titelverbindung vom Zers. p. 170°; $[\alpha]_D^{20} = -15{,}3$ (c = 1,0 in Wasser).

Das als Ausgangsmaterial benötigte Z-Trp-Ala-Ser-Gly-L-Asn-ol wird wie folgt hergestellt:

a) Z-Trp-Ala-Ser-Gly-$NHNH_2$

Zu einer Lösung von 6,0 g H-Ala-Ser-Gly-OEt, Hydrochlorid und 2,8 ml Triäthylamin in 50 ml DMF gibt man 10,5 g Z-Trp-OTcp. Nach 24 Stunden bei Raumtemperatur entfernt man das Lösungsmittel im Vakuum und trituriert den Rückstand mit verdünnter wässriger HCl und mit Essigester. Man löst den Rückstand in 80 ml DMF und fügt 12 ml Hydrazinhydrat zu. Nach 24 Stunden bei Raumtemperatur fällt man die Titelverbindung durch Zugabe von Aether aus, filtriert, wäscht mit Aethanol und trocknet.

b) Z-Trp-Ala-Ser-Gly-L-Asn-ol

Zu einer Lösung von 3,2 g Z-Trp-Ala-Ser-Gly-$NHNH_2$ in 50 ml DMF gibt man bei –20° 2,9 ml HCl-5,6N in Dioxan und 0,67 ml tert.-Butylnitrit. Nach 15 Minuten bei –20° fügt man 1,8 g L-Asparaginol, Hydrochlorid und 2,5 ml Triäthylamin zu. Nach 15 Stunden bei Raumtemperatur engt man das Reaktionsgemisch stark ein, verdünnt mit Essigester und wäscht mit verdünnter, wässriger Salzsäure und mit Wasser. Aus der konzentrierten Lösung fällt die Titelverbindung aus.

Auf analoge Weise wurden aus den entsprechenden Ausgangsverbindungen auch folgende Polypeptide der Formel I (D = Gly) hergestellt:

| Bsp. Nr. | A | B | C | E | Salzform | $[\alpha]_D^{20}$ |
|---|---|---|---|---|---|---|
| 2 | H–Trp | Ala | Ser | Asp–OH | HCl | - 1,0 [a] |
| 3 | H–Trp | Ala | Ser | Asn–OH | HCl | -16,2 [a] |
| 4 | H–Trp | Ala | Ser | Asn–OMe | HCl | -17,0 [b] |
| 5 | H–Trp | Ala | Ser | Asp–diol | HCl | -20,0 [b] |
| 6 | H–Trp | D–Ala | Ser | Asn–ol | HCl | +14,9 [a] |
| 7 | H–Trp | D–Ala | Ser | Asp–OH | HCl | - 4,6 [a] |
| 8 | H–Trp | D–Ala | Ser | Asn–OH | HCl | - 8,7 [a] |
| 9 | H–Trp | D–Ala | Ser | Asn–OMe | HCl | - 6,3 [a] |
| 10 | H–Trp | D–Ala | Ser | Asp–diol | HCl | - 7,9 [a] |
| 11 | H–D–Trp | Ala | Ser | Asn–ol | HCl | -86,3 [b] |
| 12 | H–D–Trp | Ala | Ser | Asp–OH | HCl | -75,7 [b] |
| 13 | H–D–Trp | D–Ala | Ser | Asn–ol | HCl | -72,4 [b] |
| 14 | H–D–Trp | D–Ala | Ser | Asp–OH | HCl | -10 [b] |
| 15 | H–MeTrp | Ala | Ser | Asn–ol | TFA | -10,3 [b] |
| 16 | H–MeTrp | Ala | Ser | Asp–OH | TFA | -13,5 [b] |
| 17 | H–Trp | Ala | Gly | Asp–OH | HCl | +17,6 [a] |
| 18 | H–Trp | D–Ala | Thr | Asn–ol | HCl | +18,7 [a] |
| 19 | H–Trp | D–Ala | Thr | Asp–OH | HCl | +20.9 [a] |
| 20 | H–Trp | D–Ala | Ser | Asp(OMe)–OMe | HCl | - 2,4 [a] |
| 21 | H–(5–OH)–Trp | D–Ala | Ser | Asp–OH | TFA | +27,7 [a] |
| 22 | H–Trp | Ala | Val | Asn–ol | HCl | - 1,7 [a] |
| 23 | H–Trp | D–Ala | Gly | Asp–OH | HCl | +29,8 [c] |
| 24 | H–Trp | D–Ala | Ser | Asp($NHCH_3$)–ol | HCl | +18,0 [d] |
| 25 | H–Trp | D–Ala | Val | Asp–diol | HCl | +16,7 [e] |

a: c = 1,0 in Essigsäure 95%
b: c = 1,0 in Wasser
c: c = 0,55 in Essigsäure 95%
d: c = 0,51 in Essigsäure 95%
e: c = 0,96 in Essigsäure 95%

**Patentansprüche**

1. Polypeptide bzw. Polypeptidderivate der Formel

$$A — B — C — D — E \qquad I$$

worin

A für H-Trp, H-MeTrp, H-Trp(5-OH) oder H-MeTrp(5-OH),

B für Ala,

C für Ser, Thr, Ala, Gly, Val oder einen Rest der Formel

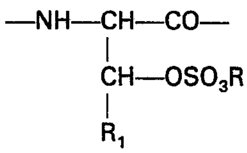

oder

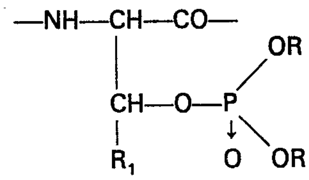

worin

R Wasserstoff, Alkyl mit 1—4 C-Atomen oder ein Alkali- oder Erdalkalimetall und

$R_1$ Wasserstoff oder Methyl bedeuten,

D für Gly oder Sar,

E für den Rest

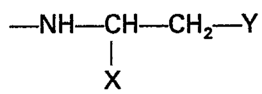

X und Y unabhängig voneinander für COZ oder $CH_2OH$,

Z für OH, $OR_2$, $NH_2$, $NHR_3$,

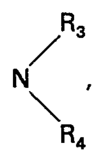,

$R_2$, $R_3$ und $R_4$ unabhängig voneinander für Alkylgruppen mit 1—4 C-Atomen stehen, wobei die Reste A, B, C und E die Konfiguration der L-, D- oder D,L-Reihe haben können, sowie Salze, Säureadditionssalze und Komplexe dieser Polypeptide bzw. Polypeptidderivate.

2. Polypeptide bzw. Polypeptidderivate der Formel

$$A' — B — C' — D — E \qquad II$$

worin

A' für H-Trp und

C' für Gly, Ser,

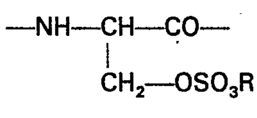

oder

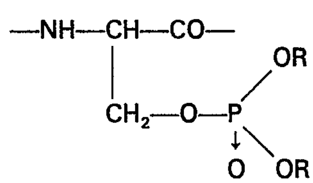

stehen und B, D und E die in Anspruch 1 angegebenen Bedeutungen haben.

3. Polypeptide bzw. Polypeptidderivate der Formel I gemäss Anspruch 1, worin A, B, C, D und E die nachfolgenden Bedeutungen haben

A H-Trp, H-MeTrp, H-Trp(5-OH) oder H-MeTrp(5-OH),

B Ala,

C Ser, Thr, Ala, Gly oder einen Rest der Formel

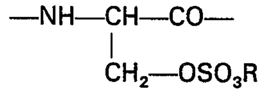

oder

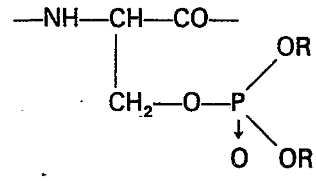

worin R für Wasserstoff, Alkyl mit 1—4 C-Atomen oder ein Alkali- oder Erdalkalimetall steht,
D Gly und
E Asp-OH, Asp-OR', Asp-$NH_2$, Asn-OH, Asn-OR', Asn-$NH_2$, Asparaginol oder den Rest

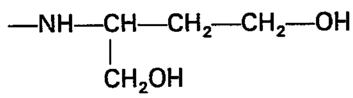

wobei R' eine Alkylgruppe mit 1—4 C-Atomen bedeutet und die Reste A, B, C und E die Konfiguration der L-, D- oder D,L-Reihe haben können.

4. Polypeptide bzw. Polypeptidderivate der Formel I gemäss Anspruch 1,
worin A, B, C, D und E die nachfolgenden Bedeutungen haben
A H-Trp, H-MeTrp, H-Trp(5-OH) oder H-MeTrp(5-OH),
B Ala,
C Ser, Thr, Ala, Gly oder einen Rest der Formel

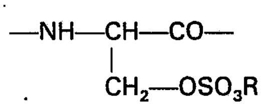

oder

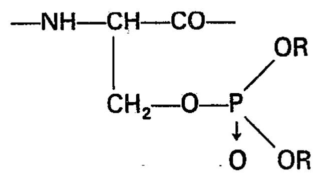

worin R Wasserstoff, Alkyl mit 1—4 C-Atomen oder ein Alkali- oder Erdalkalimetall bedeutet,
D Gly und
E Asp(OR')—X'
R' eine Alkylgruppe mit 1—4 C-Atomen und
X' eine Hydroxylgruppe, eine Alkoxygruppe mit 1—4 C-Atomen oder eine $NH_2$-Gruppe,
wobei die Reste A, B, C und Asp(OR')—X' die Konfiguration der L-, D- oder D,L-Reihe haben können.

5. Die Verbindung H-Trp-Ala-Ser-Gly-L-Asn-ol.

6. Die Verbindung H-Trp-D-Ala-Ser-Gly-Asp-diol.

7. Verfahren zur Herstellung der Verbindungen der Formel I, ihrer Salze, Säureadditionssalze und Komplexe gemäss Anspruch 1, dadurch gekennzeichnet, dass man
a) mindestens eine Schutzgruppe, die in einer geschützten Verbindung mit der in Formel I angegebenen Sequenz vorhanden ist, entfernt oder
b) zwei Peptideinheiten, von denen jede mindestens eine Aminosäure oder einen Aminoalkohol in geschützter oder ungeschützter Form enthält, durch eine Amidbindung miteinander verknüpft, wobei die Peptidbindung in der Weise erfolgen soll, dass die in der Formel I enthaltene Aminosäuresequenz hergestellt wird und anschliessend gegebenenfalls die Verfahrensstufe a) ausgeführt wird, oder
c) eine Gruppe E in eine andere Gruppe E mit der oben angeführten Definition überführt, wobei eine ungeschützte oder geschützte Verbindung der Formel I erhalten wird und in letzterem Fall der Verfahrensschritt a) ausgeführt wird.
und gewünschtenfalls die erhaltenen Verbindungen der Formel I in ihre Salze, Säureadditionssalze oder Komplexe überführt.

8. Heilmittel, enthaltend mindestens eine Verbindung gemäss Anspruch 1.

**Revendications**

1. Polypeptides ou dérivés de polypeptides de formule

$$A — B — C — D — E \qquad (I)$$

dans laquelle
A signifie H-Trp, H-MeTrp, H-Trp(5-OH) ou H-MeTrp(5-O),
B signifie Ala,
C signifie Ser, Thr, Ala, Gly, Val ou un reste de formule

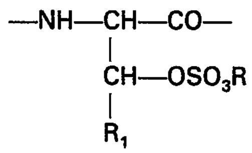

ou

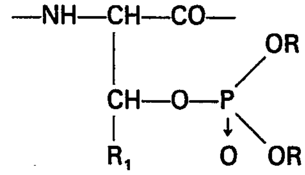

dans lesquelles R représente un atome d'hydrogène, un groupe alkyle contenant de 1 à 4 atomes de carbone ou un atome de métal alcalin ou alcalino-terreux, et $R_1$ représente un atome d'hydrogène ou un groupe méthyle,

D signifie Gly ou Ser,

E représente un reste de formule

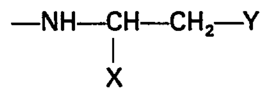

où X et Y représentent chacun, indépendamment l'un de l'autre, un groupe COZ ou $CH_2OH$,

Z représente un groupe OH, $OR_2$, $NH_2$, $NHR_3$ ou

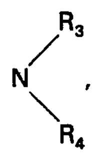

,

$R_2$, $R_3$ et $R_4$ représentent chacun, indépendamment les uns des autres, un groupe alkyle contenant de 1 à 4 atomes de carbone,

les restes A, B, C et E pouvant avoir la configuration L, D ou D,L, ainsi que les sels, les sels d'addition d'acides et les complexes de ces polypeptides ou dérivés de polypeptides.

2. Polypeptides ou dérivés de polypeptides de formule

$$A' — B — C' — D — E \qquad (II)$$

dans laquelle

A' signifie H-Trp, et

C' représente un reste Gly, Ser,

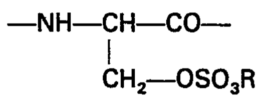

ou

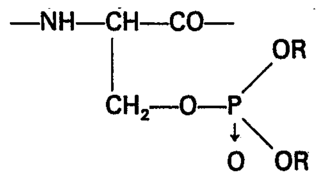

et B, D et E ont les significations données à la revendication 1.

3. Polypeptides ou dérivés de polypeptides de formule I selon la revendication 1, caractérisés en ce que A, B, C, D et E ont les significations suivantes:

A signifie H-Trp, H-MeTrp, H-Trp(5-OH) ou H-MeTrp(5-OH),

B signifie Ala,

C signifie Ser, Thr, Ala, Gly ou un reste de formule

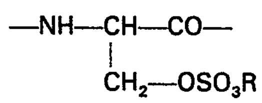

ou

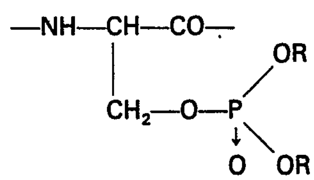

où R représente un atome d'hydrogène, un groupe alkyl contenant de 1 à 4 atomes de carbone ou un atome de métal alcalin ou alcalino-terreux,

D signifie Gly, et

E signifie Asp-OH, Asp-OR', Asp-$NH_2$, Asn-OH, Asn-OR', Asn-$NH_2$, l'asparaginol ou un reste de formule

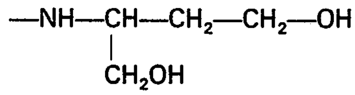

où R′ représente un groupe alkyle contenant de 1 à 4 atomes de carbone, et les restes A, B, C et E peuvent avoir la configuration L, D ou D,L.

4. Polypeptides ou dérivés de polypeptides de formule I selon la revendication 1, caractérisés en ce que A, B, C, D et E ont les significations suivantes:

A signifie H-Trp, H-MeTrp, H-Trp(5-OH) ou H-MeTrp(5-OH),

B signifie Ala,

C signifie Ser, Thr, Ala, Gly ou un reste de formule

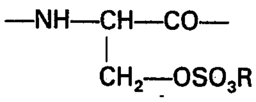

ou

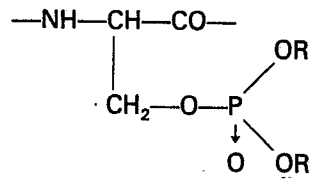

où R représente un atome d'hydrogène, un groupe alkyle contenant de 1 à 4 atomes de carbone ou un atome de métal alcalin ou alcalino-terreux,

D signifie Gly, et

E signifie Asp(OR′)-X′

où R′ représente un groupe alkyle contenant de 1 à 4 atomes de carbone, et

X′ représente un groupe hydroxy, un groupe alcoxy contenant de 1 à 4 atomes de carbone ou un groupe $NH_2$,

les restes A, B, C et Asp(OR′)-X′ pouvant avoir la configuration L, D ou D,L.

5. Le composé H-Trp-Ala-Ser-Gly-L-Asn-ol.

6. Le composé H-Trp-D-Ala-Ser-Gly-Asp-diol.

7. Un procédé de préparation des composés de formule I, de leurs sels, de leurs sels d'addition d'acides et de leurs complexes, selon la revendication 1, caractérisé en ce que

a) on élimine au moins un groupe protecteur présent dans un composé protégé comportant l'enchaînement indiqué dans la formule I, ou

b) on relie par un liaison amide deux unités peptidiques contenant chacune au moins un aminoacide ou un aminoalcool sous forme protégée ou non protégée, la liaison peptidique devant avoir lieu de manière à obtenir l'enchaînement d'aminoacides contenu dans la formule I et à pouvoir éventuellement effectuer en dernier lieu l'étape a), ou

c) on transforme un groupe E en un autre groupe E tel que défini ci-dessus, ce qui donne un composé de formule I non protégé ou protégé, ce dernier pouvant être soumis ensuite à l'étape a),

et, le cas échéant, on transforme les composés de formule I ainsi obtenus en leurs sels, en leurs sels d'addition d'acides ou en leurs complexes.

8. Un médicament, caractérisé en ce qu'il contient au moins un composé selon la revendication 1.

## Claims

1. Polypeptides or polypeptide derivatives of formula

$$A — B — C — D — E \qquad I$$

wherein

A is H-Trp, H-MeTrp, H-Trp(5-OH) or H-Me-Trp(5-OH),

B is Ala,

C is Ser, Thr, Ala, Gly, Val or a radical of formula

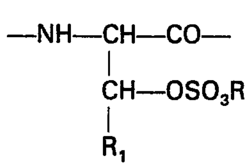

or

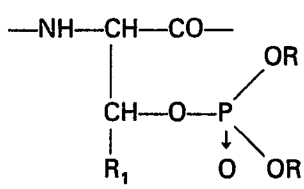

wherein R represents hydrogen, alkyl with 1—4 C-atoms or an alkali- or alkaline earth-metal and

$R_1$ represents hydrogen or methyl,

D is Gly or Sar,

E is the radical

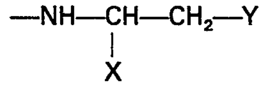

X and Y independently of each other are COZ or $CH_2OH$,
Z is OH, $OR_2$, $NH_2$, $NHR_3$,

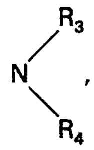,

$R_2$, $R_3$ and $R_4$ independently of each other are alkyl groups with 1—4 C-atoms, whereby the radicals A, B, C and E can have the configuration of the L-, D- or D,L-series, as well as salts, acid addition salts and complexes of these polypeptides or polypeptide derivatives.

2. Polypeptides or polypeptide derivatives of formula

$$A' — B — C' — D — E \qquad II$$

wherein
A′ is H-Trp and
C′ is Gly, Ser,

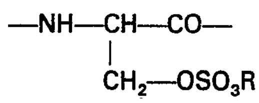

or

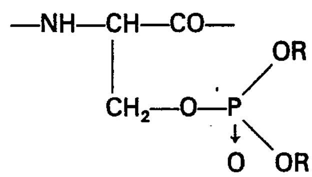

and B, D and E have the meanings given in claim 1.

3. Polypeptides or polypeptide derivatives of formula I according to claim 1, wherein A, B, C, D and E have the following meanings
A H-Trp, H-MeTrp, H-Trp(5-OH) or H-MeTrp(5-OH),
B Ala,
C Ser, Thr, Ala, Gly or a radical of formula

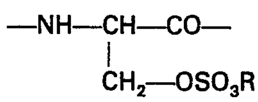

or

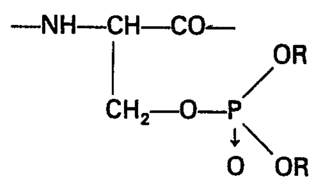

wherein R is hydrogen, alkyl with 1—4 C-atoms or an alkali- or alkaline earth-metal,
D Gly and
E Asp-Oh, Asp-OR′, Asp-$NH_2$, Asn-OH, Asn-OR′, Asn-$NH_2$, asparaginol or the radical

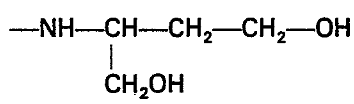

whereby R′ represents an alkyl group with 1—4 C-atoms and the radicals, A, B, C and E can have the configuration of the L-, D- or D,L-series.

4. Polypeptides or polypeptide derivatives of formula I according to claim 1, wherein A, B, C, D and E have the following meanings
A H-Trp, H-MeTrp, H-Trp(5-OH) or H-MeTrp(5-OH),
B Ala,
C Ser, Thr, Ala, Gly or a radical of formula

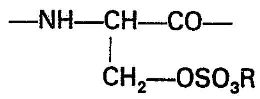 or 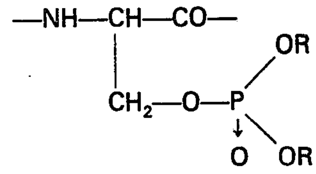

wherein R represents hydrogen, alkyl with 1—4 C-atoms or an alkali- or alkaline earth-metal,

D Gly and

E Asp(OR')-X'

R' an alkyl group with 1—4 C-atoms and

X' a hydroxy group, an alkoxy group with 1—4 C-atoms or an $NH_2$-group;

whereby the radicals A, B, C and Asp(OR')-X' can have the configuration of the L-, D- or D,L-series.

5. The compound H-Trp-Ala-Ser-Gly-L-Asn-ol.

6. The compound H-Trp-D-Ala-Ser-Gly-Asp-diol.

7. Process for the production of compounds of formula I, their salts, acid addition salts and complexes according to claim 1, characterised by

a) removing at least one protecting group present in a protected compound having the sequence given in formula I, or

b) linking together via an amide bond two peptide units, each of which contains at least one amino acid or an amino-alcohol in protected or unprotected form, whereby the peptide bonding occurs such that the amino acid sequence contained in formula I is obtained, and then optionally carrying out process step a), or

c) converting one group E into another group E having the meaning given above, whereby an unprotected or protected compound of formula I is obtained and, in the latter case, carrying out process step a).

and optionally converting the compound of formula I obtained into its salts, acid addition salts or complexes.

8. Medicament containing at least one compound according to claim 1.